# EUROPÄISCHE PATENTSCHRIFT

(11) **EP 1 645 246 B1**
(45) Veröffentlichungstag und Bekanntmachung des Hinweises auf die Patenterteilung: **16.03.2011**
(21) Anmeldenummer: 05019043.8
(22) Anmeldetag: 01.09.2005
(51) Int. Cl.: A61F 2/90, A61F 2/92

(54) **Stentsystem**
Stent system
Système de stent

(30) Priorität: 05.10.2004 DE 102004048458
(43) Veröffentlichungstag der Anmeldung: 12.04.2006
(73) Patentinhaber: Admedes Schuessler GmbH, 75179 Pforzheim (DE)
(72) Erfinder: Flaxmeier, Erik, 76307 Karlsbad (DE); Strobel, Martin, 76131 Karlsruhe (DE); Steiner, Ralf, 75181 Pforzheim (DE); Mailänder, Werner, 75217 Birkenfeld (DE); Schüssler, Andreas, 76327 Pfinztal (DE)
(74) Vertreter: Müller-Boré & Partner Patentanwälte

(56) Entgegenhaltungen:
- WO-A-02/055125
- DE-A1- 10 301 600
- US-A- 5 064 435
- US-A1- 2003 212 450
- US-B1- 6 325 823
- US-B1- 6 585 758
- US-B1- 6 613 078

## Beschreibung

Die vorliegende Erfindung betrifft ein Stentsystem zum Implantieren in einen lebenden Korper, insbesondere als Aneurysmen-Stentsystem bzw. Endoprothese fur intrazerebrale Gefäßaussackungen, sog. Aneurysmen.

Interzerebrale Gefäßaussackungen -sog. Aneurysmen- sind die häufigste Ursache der nicht-traumatischen Subarachnoidalblutung. Ihre Inzidenz liegt in der Gesamtbevolkerung bei 1% nach Autopsiestudien sogar bis zu 9%. Pathomorphologisch sind intrazerebrale Aneurysmen in der Regel echte, sakkulare Aneurysmen, die meist in Gefäßaufzweigungen lokalisiert sind (Vgl. z.B. Schumacher M."Diagnostic workup in cerebral aneurysms" in Nakstadt PHj (ed):"cerebral aneurysms", pp13-24, Bologna: Centauro (2000)).

EP 0737452 beschreibt einen Stent mit zusätzlich eingewobenen Fäden, um die Fluiddurchlässigkeit an einem Abschnitt des Stents zu verringern.

WO 99/02092 beschreibt einen Aneurysmen Stent mit einem ersten und einem zweiten Abschnitt, wobei der zweite Abschnitt eine verringerte Fluiddurchlässigkeit aufweist. Der zweite Abschnitt befindet sich bei diesem Stent nur in einem begrenzten Bereich des Umfangs, der im Bereich des Aneurysmus anzuordnen ist.

DE 103 01 600 A1 beschreibt eine Kombination von Stents, bei der ein zweiter Stent innerhalb eines ersten Stents angeordnet ist, so daß die Überlagerung der Gitter- bzw. Maschenstruktur eine erhöhte Mantelflächendichte ergibt.

US-B1-6 613 078 beschreibt ein endoluminales Mehrkomponenten-Implantat mit einem Rahmenstent und mehreren daran fix befestigten Stents.

Die Aufgabe der vorliegenden Erfindung besteht in der Schaffung eines verbesserten Aneurysmen Stents, der einfach herzustellen ist, einfach implantiert werden kann und eine gute Röntgensichtbarkeit aufweist.

Diese Aufgabe wird durch einen Aneurysmen-Stent nach Anspruch 1 und 5 gelöst. Bevorzugte Ausführungsbeispiele sind in den abhängigen Ansprüchen angeführt.

Gemäß der Erfindung wird ein Aneurysma-Stentsystem fur interzerebrale Gefäßaussackungen zum Implantieren in einen lebenden Körper bereitgestellt, das zwei Stents bzw. Geflechte aufweist. Die beiden Geflechte haben entweder unterschiedliche oder auch gleichartige Maschenweiten oder Maschenstrukturen, so dass ein Bereich der Überlagerung der beiden Geflechte einen Bereich mit verringerter Blutdurchlässigkeit ergibt, welcher an bzw. in der Umgebung eines Aneurysmahalses anordenbar ist.

Durch die veränderte Blutdurchlässigkeit (durch die Kombination der beiden Stentstrukturen) wird eine Strömung durch die beiden Stentwände so beeinflusst, dass eine Durchsatzmenge in das Aneurysma hinein begrenzt wird. Diese Durchflussbegrenzung führt zu einer Thrombogenierung des Blutes im Aneurysma und damit zur Verödung des Aneurysmas.

Das komplette Stentsystem ist komprimierbar, so dass es in ein Zuführsystem eingebracht werden kann. Das Stentsystem ist selbstexpandierend, so dass das System nach Ausbringen aus dem Zuführsystem selbständig expandiert.

Der Aneurysmen-Stent wird in einen lebenden Körper implantiert, insbesondere zur Behandlung von Aneurysmen, und hat ein im wesentlichen rohrförmiges erstes Geflecht und ein im wesentlichen rohrförmiges zweites Geflecht, wobei das zweite Geflecht eine geringere Durchlässigkeit als das erste Geflecht aufweist und eines der beiden Geflechte auf das andere aufgeschoben oder in das andere eingeschoben ist, so dass die beiden Geflechte koaxial zueinander angeordnet sind, wobei beide Geflechte aneinander befestigt sind, so dass diese im kollabierten Zustand gemeinsam in den lebenden Körper implantierbar sind.

Indem eines der beiden Geflechte eine geringere Fluiddurchlässigkeit aufweist, wird eine Thrombogenisierung unterstützt und ein Platzen des Aneursymas verhindert. Darüber hinaus wird die Herstellung eines Bereichs mit verringerter Fluiddurchlässigkeit dadurch vereinfacht, dass zwei Geflechte mit unterschiedlicher Fluiddurchlässigkeit ineinandergeschoben werden, um koaxial angeordnet zu sein. Ein Drehen des eingeführten Stents erübrigt sich, da der Bereich mit verringerter Fluiddurchlässigkeit am gesamten Umfang des Stents vorhanden ist.

Vorzugsweise ist die Kontur zumindest eines der Geflechte so gestaltet, dass ein Formschluss in axialer Richtung zwischen den beiden Geflechten entsteht, so dass ein Verrutschen bzw. axiales Verlagern verhindert wird. Hierdurch können beide Geflechte bzw. Stents gleichzeitig in den Körper eingeführt werden.

Vorzugsweise weist das erste Geflecht einen Bereich mit vergrößertem Durchmesser auf, in dem das zweite Geflecht aufgenommen ist, so dass ein Innendurchmesser des zweiten Geflechts im wesentlichen gleich einem Innendurchmesser des ersten Geflechts in einem Bereich mit nicht vergrößertem Durchmesser ist. Hierdurch wird verhindert, dass im Übergang eine Abstufung entsteht, so dass das Strömungsverhalten am Übergang nicht beeinträchtigt ist.

Vorzugsweise weist das erste Geflecht einen Bereich mit verringertem Durchmesser auf, auf den das zweite Geflecht aufgepaßt ist, so dass ein Außendurchmesser des zweiten Geflechts im wesentlichen gleich einem Außendurchmesser des ersten Geflechts in einem Bereich mit nicht verringertem Durchmesser ist. Hierdurch wird ein Einführen der beiden Geflechte erleichtert und es wird eine flächige Anlage an dem Körpergewebe erzielt.

Alternativ weist das zweite Geflecht eine Folie auf, die im kontrahierten Zustand des ersten Geflechts spiralförmig auf das erste Geflecht aufgewickelt ist und im expandierten Zustand des ersten Geflechts so abgewickelt ist, dass die Außenumfangsfläche des ersten Geflechts im wesentlichen vollständig bedeckt ist.

Vorzugsweise sind die beiden Geflechte durch zumindest einen Schweißpunkt aneinander befestigt und eventuelle weitere Schweißpunkte liegen auf einer Umfangslinie der rohrförmigen Geflechte, vorzugsweise in einer axialen Mitte der Geflechte. Hierdurch werden Bewegungen des inneren Geflechts innerhalb des äußeren Geflechts ermöglicht, während eine axiale Bewegung und ein Verkanten des inneren Geflechts verhindert wird.

Vorzugsweise besteht das innenliegende Geflecht aus einem Formgedächtnismetall und erzeugt eine hohe Radialkraft, wobei das äußere Geflecht keine eigene Aufstellkraft hat.

Vorzugsweise weist eines der Geflechte Haken auf zum in Eingriff treten mit dem anderen Geflecht, um eine Fixierung der beiden Geflechte zu erzielen.

Der erfindungsgemäße Aneurysmen-Stent wird vorzugsweise als ballonexpandierter Aneurysmen-Stent verwendet.

Die Erfindung wird anhand bevorzugter Ausführungsbeispiele nachfolgend näher erläutert unter Bezugnahme auf die Zeichnungen.

Fig. 1 zeigt ein erstes Ausführungsbeispiel eines erfindungsgemäßen Aneurysmen-Stents.

Fig. 2 zeigt ein zweites Ausführungsbeispiel eines erfindungsgemäßen Aneurysmen-Stents.

Fig. 3 zeigt ein drittes Ausführungsbeispiel eines erfindungsgemäßen Aneurysmen-Stents im expandierten Zustand.

Fig. 4 zeigt den in Fig. 3 gezeigten Aneurysmen-Stent im kollabierten bzw. komprimierten Zustand.

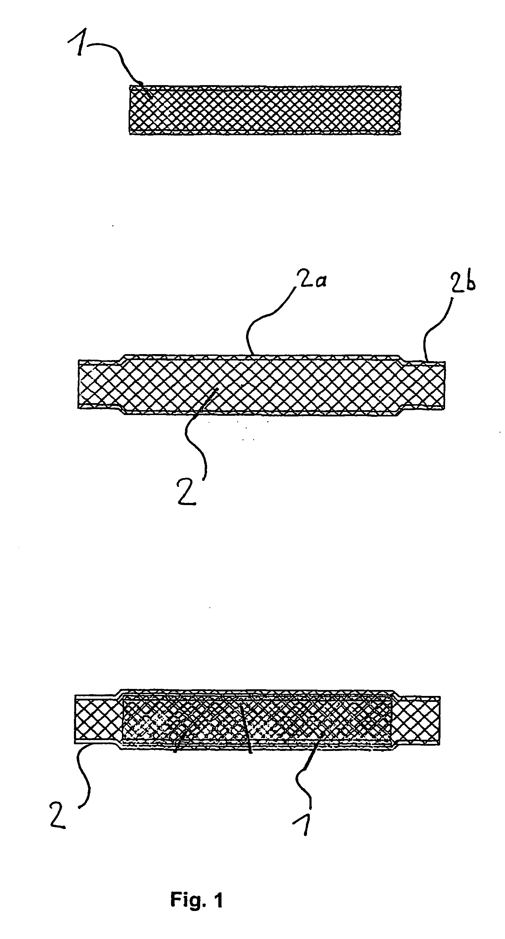

Wie in Fig. 1 gezeigt ist, weist ein erfindungsgemäßer Aneurysmen-Stent nach dem ersten Ausführungsbeispiel ein inneres Geflecht 1 und ein äußeres Geflecht 2 auf. Das äußere Geflecht 2 bzw. der äußere Stent 2 ist so ausgebildet, daß es in einem mittleren Bereich einen Bereich 2a mit vergrößertem Durchmesser gibt. Das innere Geflecht 1 bzw. der innere Stent 1 kann formschlüssig in diesen Bereich 2a mit vergrößertem Durchmesser des Geflechts 2 eingepaßt werden. Durch die formschlüssige Verbindung wird ein Verrutschen der beiden Geflechte 1, 2 relativ zueinander verhindert. Darüber hinaus ist der aufgeweitete Durchmesser des Geflechts 2 so dimensioniert, daß es keine Stufe bzw. keinen Durchmesserübergang gibt, wodurch ein Strömungsverhalten des Stents so verbessert ist, daß eine Thromboseneigung reduziert wird, wenn der Stent in einen Körper eingesetzt ist.

Das innere Geflecht 1 wird an dem äußeren Geflecht 2 über einen oder zwei Schweißpunkte befestigt, die sich auf einer Umfangslinie des Stents befinden. Hierdurch ist eine Bewegung des inneren Geflechts 1 innerhalb des äußeren Geflechts möglich. Durch die Fixierung der beiden Geflechte 1, 2 aneinander ist es möglich, daß die Baugruppe bestehend aus den beiden Geflechten 1, 2 gemeinsam in ein Einführsystem, beispielsweise einen Katheter geladen wird, um gemeinsam in einen Körper eingeführt zu werden und gemeinsam expandiert zu werden.

Alternativ oder zusätzlich wird das innere Geflecht 1 mittels Haken an dem äußeren Geflecht 2 befestigt.

Das innere Geflecht 1 weist im Vergleich zu dem äußeren Geflecht 2 eine verringerte Durchlässigkeit auf, die durch eine erhöhte Materialanhäufung erreicht werden kann. So kann das innere Geflecht 1 z.B. eine im Vergleich zu dem äußeren Geflecht 2 erhöhte Anzahl an Streben pro Fläche aufweisen, d.h. engmaschiger ausgebildet sein. Alternativ oder zusätzlich kann die Wandung des inneren Geflechts 1 eine geringere Porosität und/oder Durchgangsöffnungen mit geringerem Durchmesser bzw. Fläche aufweisen. Zumindest eines der Geflechte 1, 2 weist bevorzugt eine Geometrie entsprechend der Anmeldung DE 102 28 529.2 bzw. PCT/EP03/06775 oder DE 103 23 475.6 auf. Das äußere Geflecht 2 ist bevorzugt derart expandiert, daß sie an einer Wandung des Blutgefäßes bevorzugt flächig anliegt und dieses abstützt. Das innere Geflecht 1 ist bevorzugt derart innerhalb des äußeren Geflechts 2 angeordnet, daß es bei Verwendung zumindest teilweise einem Aneurismen-Hals entspricht bzw. diesen abdeckt. Somit kann lediglich ein verminderter Blutstrom in das Aneurisma eintreten und somit eine Thrombogeneisierung unterstützt werden bzw. ein Platzen des Aneurisma verhindert werden.

Für die Expansion besteht der äußere Stent 2 aus einem Formgedächtnismetall, beispielsweise aus Nitinol. Durch die Fixierung des inneren Geflechts 1 an dem äußeren Geflecht 2 wird das innere Geflecht 1 mitexpandiert, wenn das äußere Geflecht 2 expandiert, so daß das innere Geflecht 1 aus einem röntgensichtbaren Material wie beispielsweise Tantal, Platin, Platin-Iridium oder Gold gefertigt werden kann.

In dem Bereich der Überlappung der beiden Geflechte 1, 2 ergibt sich eine verringerte Fluiddurchlässigkeit bzw. Blutdurchlässigkeit. Dieser überlappende Bereich ist beim Implantieren des Stents im Bereich des Aneurysmas anzuordnen, um die gewünschte Wirkung der verringerten Blutdurchlässigkeit zu erzielen. Da der überlappende Bereich im gesamten Umfangsbereich des Stents angeordnet ist, erübrigt sich ein Drehen des eingeführten Stents, um den Bereich mit verringerter Blutdurchlässigkeit bei dem Aneurisma anzuordnen. Hierdurch wird der Einführvorgang erleichtert und das Verletzungsrisiko herabgesetzt.

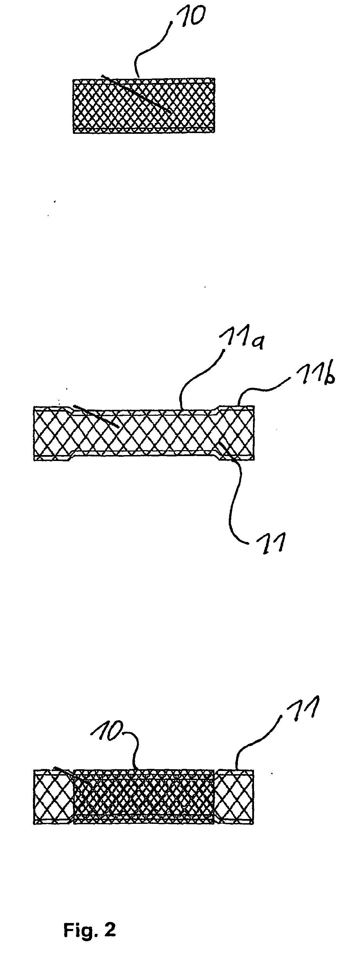

Ein zweites Ausführungsbeispiel wird unter Bezugnahme auf Fig. 2 beschrieben. Bei dem zweiten Ausführungsbeispiel hat ein Geflecht 11 einen Bereich 11a mit verringertem Durchmesser. Auf diesen Bereich 11a mit verringertem Durchmesser des Geflechts 11 wird ein Geflecht 10 aufgepaßt, so daß ein Stent 10, 11 mit gleichbleibendem Außendurchmesser entsteht. Der Innendurchmesser des Stents 10, 11 ist in dem überlappenden Bereich etwas verringert. In dem überlappenden Bereich der Geflechte 10, 11 ergibt sich wieder die verringerte Fluiddurchlässigkeit bzw. Blutdurchlässigkeit, wie bei dem ersten Ausführungsbeispiel. Der Außendurchmesser des äußeren Geflechts 10 ist im wesentlichen derselbe wie der des inneren Geflechts 11 in dem Bereich mit nicht verringertem Durchmesser 11b. Hierdurch wird ein stufiger Übergang zwischen den beiden Geflechten vermieden, wodurch das Einführen des Stents 10, 11 erleichtert wird. Außerdem liegt der Stent 10, 11 im expandierten Zustand flächig an der Gewebewandung an. Die Wirkung und das Implantieren dieses Stents 10, 11 ist dasselbe wie bei dem ersten Ausführungsbeispiel und wird deshalb nicht wiederholt erläutert.

Der innere Stent 11 ist aus einem Formgedächtnismetall wie beispielsweise Nitinol hergestellt und hat einen hohe Radialkraft bei der Expansion, so daß der äußere Stent 10 durch die hohe Radialkraft des inneren Stents 11 expandiert wird. Der äußere Stent 10 ist aus einem sehr dünnen röntgensichtbaren Material hergestellt und hat keine eigene Expansionskraft.

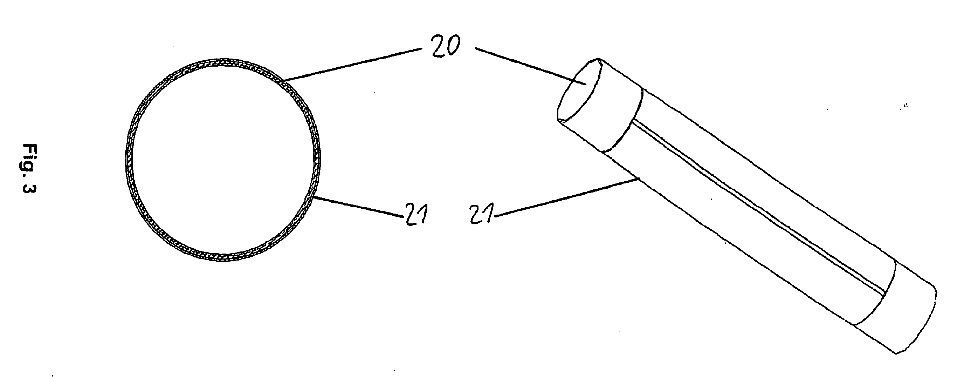

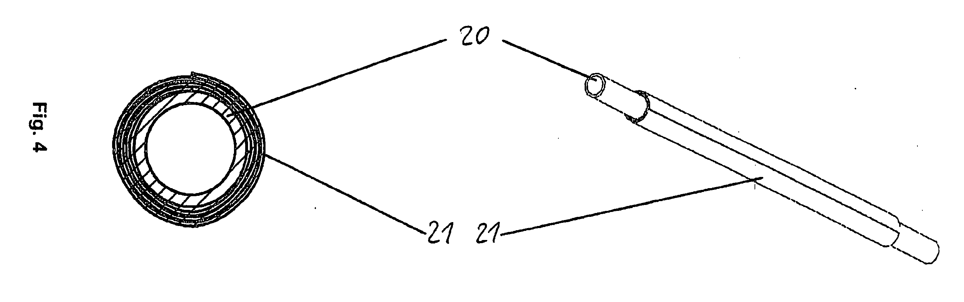

Unter Bezugnahme auf die Figs. 3 und 4 wird ein drittes Ausführungsbeispiel der vorliegenden Erfindung erläutert. Fig. 3 zeigt einen Aneurismen-Stent des dritten Ausführungsbeispiels im expandierten Zustand, während Fig. 4 denselben Aneurismen-Stent im kontrahierten Zustand zeigt.

Das Geflecht 20 ist ähnlich dem Geflecht 11 des zweiten Ausführungsbeispiels aufgebaut, d.h. es hat einen Bereich mit verringertem Durchmesser. Auf diesen Bereich mit verringertem Durchmesser des Geflechts 20 ist jedoch eine Folie 21 aufgewickelt, wie am besten in Fig. 4 (unten) gezeigt ist. Diese Folie 21 ist an einem ihrer Enden an dem Geflecht 20 befestigt und spiralförmig auf das Geflecht 20 aufgewickelt. Bei der Expansion des Geflechts 20 wird die Folie 21 so abgewickelt, daß sie im wesentlichen den Außenumfang des durchmesserverringerten Bereichs des Geflechts 20 umgibt, wie in Fig. 3 gezeigt ist. Das innere Geflecht 20 ist aus einem Formgedächtnismetall wie beispielsweise Nitinol hergestellt. Die Folie ist aus Tantal, Platin-Iridium oder Nitinol hergestellt.

Die Erfindung ist nicht auf die hier beschriebenen Ausführungsbeispiele beschränkt, sondern kann beliebig abgewandelt werden. Beispielsweise kann der Stent 20 des dritten Ausführungsbeispiels einen gleichförmigen Durchmesser haben. Außerdem können die beiden Geflechte des ersten und zweiten Ausführungsbeispiels auch noch durch weitere Befestigungsmittel aneinander befestigt werden. Neben dem genannten Befestigungsmittel Schweißpunkte und Haken der Geflechte sind hier noch das Verflechten mit zusätzlichen Fäden und das Verkleben der beiden Geflechte zu nennen.

Die Ausführungsbeispiele können darüber hinaus beliebig kombiniert werden.

Die beschriebenen Stents können zumindest bereichsweise mit einer thrombogenen Beschichtung z.B. aus Platin oder einem anderen Metall oder Arzneistoff versehen sein. Weiterhin können die Stents bevorzugt als "drug eluding stents" ausgebildet sein. Weiterhin können die Stents vorteilhaft eingesetzt werden, um ein Kollabieren des Gefäßes bzw. Hohlraumes zu verhindern und die Wandung(en) abzustützen.

### Bezugszeichenliste

- 1: Innengeflecht
- 2: Außengeflecht
- 2a: Bereich mit vergrößertem Durchmesser
- 2b: Bereich mit verringertem Durchmesser
- 10: Außengeflecht
- 11: Innengeflecht
- 11a: Bereich mit verringertem Durchmesser
- 11b: Bereich mit verringertem Durchmesser
- 20: Innengeflecht
- 21: Folie

## Patentansprüche

1. Aneurysmen-Stent zum Implantieren in einen lebenden Körper, insbesondere zur Behandlung von Aneurysmen, mit einem im wesentlichen rohrförmigen ersten Geflecht (2; 11; 20) und einem im wesentlichen rohrförmigen zweiten Geflecht (1; 10; 21), wobei das zweite Geflecht (1; 10; 21) eine geringere Durchlässigkeit als das erste Geflecht (2; 11; 20) aufweist und eines der beiden Geflechte auf das andere aufgeschoben oder in das andere eingeschoben ist, so dass die beiden Geflechte koaxial zueinander angeordnet sind, wobei beide Geflechte aneinander befestigt sind, so dass diese im kollabierten Zustand gemeinsam in den lebenden Körper implantierbar sind,
**dadurch gekennzeichnet, dass**
eines der Geflechte aus einem röntgensichtbaren Material wie beispielsweise Tantal, Platin, Platin-Iridium oder Gold gefertigt ist und keine eigene Expansionskraft hat, und das andere aus einem Formgedächtnismetall hergestellt ist, wobei die beiden Geflechte aufgrund der Fixierung aneinander gleichzeitig expandierbar sind.

2. Aneurysmen-Stent nach Anspruch 1, wobei die Kontur zumindest eines der Geflechte so gestaltet ist, dass ein Formschluss in axialer Richtung zwischen den beiden Geflechten entsteht, so dass ein axiales Verlagern verhindert wird.

3. Aneurysmen-Stent nach Anspruch 1 oder 2, wobei das erste Geflecht (2) einen Bereich (2a) mit vergrößertem Durchmesser aufweist, in dem das zweite Geflecht (1) aufgenommen ist, so dass ein Innendurchmesser des zweiten Geflechts (1) im wesentlichen gleich einem Innendurchmesser des ersten Geflechts (2) in einem Bereich (2b) mit nicht vergrößertem Durchmesser ist.

4. Aneurysmen-Stent nach Anspruch 1 oder 2, wobei das erste Geflecht (11) einen Bereich (11a) mit verringertem Durchmesser aufweist, auf den das zweite Geflecht (10) aufgepaßt ist, so dass ein Außendurchmesser des zweiten Geflechts (10) im wesentlichen gleich einem Außendurchmesser des ersten Geflechts (11) in einem Bereich (11b) mit nicht verringertem Durchmesser ist.

5. Aneurysmen-Stent zum Implantieren in einen lebenden Körper, insbesondere zur Behandlung von Aneurysmen, mit einem im wesentlichen rohrförmigen ersten Geflecht (20) und einer Folie (21), die im kontrahierten Zustand des ersten Geflechts (20) spiralförmig auf das erste Geflecht (20) aufgewickelt ist und im expandierten Zustand des ersten Geflechts (20) so abgewickelt ist, dass die Außenumfangsfläche des ersten Geflechts (20) im wesentlichen vollständig bedeckt ist, wobei das erste Geflecht (20) aus einem Formgedächtnismetall hergestellt ist und die Folie (21) an einem ihrer Enden an dem ersten Geflecht (20) befestigt ist, so dass die Folie (21) im aufgewickelten Zustand gemeinsam mit dem ersten Geflecht (20) im kollabierten Zustand in den lebenden Körper implantierbar ist, und die Folie aus einem röntgensichtbaren Material wie beispielsweise Tantal oder Platin-Iridium gefertigt ist.

6. Aneurysmen-Stent nach einem der vorherigen Ansprüche 1 bis 4, wobei die beiden Geflechte durch zumindest einen Schweißpunkt aneinander befestigt sind und eventuelle weitere Schweißpunkte auf einer Linie am Umfang mittig der rohrförmigen Geflechte liegen.

7. Aneurysmen-Stent nach einem der vorherigen Ansprüche1 bis 4, wobei das innenliegende Geflecht aus einem Formgedächtnismetall besteht und eine hohe Radialkraft erzeugt und das äußere Geflecht keine eigene Aufstellkraft hat.

8. Aneurysmen-Stent nach einem der vorherigen Ansprüche1 bis 4, wobei eines der Geflechte Haken aufweist zum in Eingriff treten mit dem anderen Geflecht, um eine Fixierung der beiden Geflechte zu erzielen.

## Claims

1. Aneurysm stent for implanting in a living body, in particular for treating aneurysms, having a substantially tubular first mesh device (2; 11; 20) and a substantially tubular second mesh device (1; 10; 21), wherein the second mesh device (1; 10; 21) comprises a reduced permeability in comparison to the first mesh device (2; 11; 20) and one of the two mesh devices is pushed onto the other or inserted into the other mesh device, so that the two mesh devices are disposed coaxially with respect to each other, wherein two mesh devices are attached to each other, so that these can be implanted in the collapsed state conjointly into the living body, **characterised in that**:
one of the mesh devices is produced in an X-ray visible material such as for example, tantalum, platinum, platinum-iridium or gold and has no innate expansion force, and the other mesh device is produced from a shape-memory metal, wherein the two mesh devices can expand simultaneously owing to their being fixed to each other.

2. Aneurysm stent as claimed in claim 1, wherein the contour at least of one of the mesh devices is formed in such a manner that a positive fit in the axial direction between the two mesh devices is created, so that an axial shifting from position is prevented.

3. Aneurysm stent as claimed in claim 1 or 2, wherein the first mesh device (2) comprises a region (2a) having an enlarged diameter which receives the second mesh device (1), so that an inner diameter of the second mesh device (1) is substantially equal to an inner diameter of the first mesh device (2) in a region (2b) which does not have an enlarged diameter.

4. Aneurysm stent as claimed in claim 1 or 2, wherein the first mesh device (11) comprises a region (11a) having a reduced diameter onto which the second mesh device (10) is fitted, so that an outer diameter of the second mesh device (10) is substantially equal to an outer diameter of the first mesh device (11) in a region (11b) which does not have a reduced diameter.

5. Aneurysm stent for implanting in a living body, in particular for treating aneurysms, having a substantially tubular first mesh device (20) and a film (21) which in the contracted state of the first mesh device (20) is wound as a spiral onto the first mesh device (20) and in the expanded state of the first mesh device (20) is unwound in such a manner that the outer peripheral surface of the first mesh device (20) is substantially fully covered, wherein the first mesh device (20) is produced from a shape-memory metal and the film (21) is attached at one of its ends to the first mesh device (20), so that the film (21) in the wound-up state can be implanted conjointly with the first mesh device (20) in the collapsed state into the living body, and the film is produced from an X-ray-visible material such as for example tantalum or platinum-iridium.

6. Aneurysm stent as claimed in any one of the preceding claims 1 to 4, wherein the two mesh devices are attached to each other by at least one weld spot and possible further weld spots lie on a line on the periphery in the middle of the tubular mesh devices.

7. Aneurysm stent as claimed in any one of the preceding claims 1 to 4, wherein the innerlying mesh device consists of a shape-memory metal and generates a high radial force and the outer mesh device has no innate positioning force.

8. Aneurysm stent as claimed in any one of the preceding claims 1 to 4, wherein one of the mesh devices comprises hooks for engaging with the other mesh device in order to fix the two mesh devices.

## Revendications

1. Système de stent pour anévrismes à implanter dans un organisme vivant, en particulier pour le traitement des anévrismes, avec un premier treillis essentiellement tubulaire (2 ; 11 ; 20) et un second treillis essentiellement tubulaire (1 ; 10 ; 21), dans lequel le second treillis (2; 11 ; 20) présente une perméabilité inférieur à celle du premier treillis, où l'un des deux treillis soit se glisse à l'intérieur de l'autre, soit recouvre l'autre, de sorte que les deux treillis soient orientés dans le même axe, où les deux treillis sont fixés l'un à l'autre, de sorte que ceux-ci, en état affaissé puissent, être implantés ensemble dans un organisme vivant,
**caractérisé en ce que**
un des treillis est fabriqué dans un matériau visible aux rayons X comme par exemple le tantale, le platine, le platine-iridium ou l'or, et ne possède pas de pouvoir expansif propre, et que l'autre est fabriqué dans un métal à mémoire de forme, dans lequel les deux treillis sont, en raison de leur fixation mutuelle, expansibles en même temps.

2. Système de stent pour anévrismes selon la revendication 1, dans lequel le profil d'au moins un des treillis est conformé de manière à créer une fermeture géométrique, dans la direction axiale, entre les deux treillis, de sorte qu'un déplacement axial soit évité.

3. Système de stent pour anévrismes selon la revendication 1 ou 2, dans lequel le premier treillis (2) présente une zone (2a) dont le diamètre est supérieur, dans laquelle le second treillis (1) se glisse, de sorte qu'un diamètre intérieur du second treillis (1) soit essentiellement identique au diamètre intérieur du premier treillis (2) dans une zone (2b) avec un diamètre non agrandi.

4. Système de stent pour anévrismes selon la revendication 1 ou 2, dans lequel le premier treillis (11) présente une zone (11a) dont le diamètre est inférieur, sur laquelle le second treillis (10) est ajusté, de sorte qu'un diamètre extérieur du second treillis (10) soit essentiellement identique au diamètre extérieur du premier treillis (11) dans une zone (11b) avec un diamètre non réduit.

5. Système de stent pour anévrismes à implanter dans un organisme vivant, en particulier pour le traitement des anévrismes, avec un premier treillis essentiellement tubulaire (20) et une feuille (21) qui, lorsque le premier treillis est en état contracté (20), est enroulée en spirale autour du premier treillis (20), et qui, lorsque le premier treillis est en état dilaté, est déroulée de telle sorte que la surface extérieure du premier treillis (20) soit essentiellement complètement recouverte, dans lequel le premier treillis (20) est fabriqué dans un métal à mémoire de forme et la feuille (21), à une de ses extrémités, est fixée au premier treillis (20), de sorte que la feuille (21), en état enroulé, puisse être implantée dans un organisme vivant avec le premier treillis (20) en état affaissé, et la feuille est fabriquée dans un matériau visible aux rayons X comme le tantale ou le platine-iridium.

6. Système de stent pour anévrismes selon l'une quelconque des revendications précédentes 1 à 4, dans lequel les deux treillis sont fixés entre eux par au moins un point de soudure et d'éventuels autres points de soudure situés sur une ligne axiale des treillis tubulaires.

7. Système de stent pour anévrismes selon l'une quelconque des revendications précédentes 1 à 4, dans lequel le treillis intérieur est composé d'un métal à mémoire de forme et produit une force radiale élevée, et le treillis extérieur ne possède aucune force de redressement propre.

8. Système de stent pour anévrismes selon l'une quelconque des revendications précédentes 1 à 4, dans lequel un des treillis présente des crochets qui s'engrènent sur l'autre treillis afin d'obtenir une fixation des deux treillis.
